# EUROPEAN PATENT APPLICATION

(11) **EP 2 703 015 A1**
(43) Date of publication of application: **05.03.2014**
(21) Application number: 12006144.5
(22) Date of filing: 29.08.2012
(51) Int. Cl.: A61L 27/12, A61L 27/24, A61L 27/46, A61L 27/58, A61L 27/38

(54) **Bioresorbable membrane**

(71) Applicant: Straumann Holding AG, 4002 Basel (CH)
(72) Inventor: The designation of the inventor has not yet been filed
(74) Representative: Schaad, Balass, Menzl & Partner AG

(57) **Abstract**

The preset invention relates to a bioresorbable membrane having a first surface area, an opposite second surface area and an inner part arranged between said first surface area and said second surface area. The first surface area is intended to be directed towards a defect site. The membrane comprises cross-linked collagen fibers obtained from gelatin. A strontium ion containing osteoconductive powder material is dispersed in at least said first surface area resulting in a membrane which stimulates bone formation at early stages.

## Description

The present invention relates to bioresorbable membranes comprising cross-linked collagen fibers obtained from gelatin and to a method for producing the same. Said membranes are useful for medical applications, such as guided bone or tissue regeneration.

Dental implants that are used for insertion into bone, for example titanium screws to be placed into the jaw for attachment of artificial teeth, are known per se. The function of such an implant can be hampered by an insufficient bone volume or the presence of bone defects at the site of implantation.

An often applied measure to promote bone formation at the implantation site is guided bone regeneration (GBR). In this procedure, the site where bone formation is desired is separated from the surrounding soft tissue by a barrier membrane that inhibits non-osteogenic soft tissue cells from entering the site, thus allowing bone forming cells derived from the bone marrow to fill it with bone.

There are several types of membranes that are used in the field of guided bone or tissue regeneration in general. Typical procedures involve the implantation of a membrane. The regeneration may take place through the recruitment of cells from the surrounding tissue towards the biomaterial structure.

US 2006/0149392 discloses a membrane comprising a biodegradable scaffold and at least one growth factor chemically immobilized to the biodegradable scaffold. Said growth factor also promotes the development of bone or cartilage.

EP 1 574 229 discloses a medical film, wherein a reinforcing material made of a biodegradable polymer is placed in a gelatin solution so as to allow the solution to infiltrate in the reinforcing material. The reinforcing material may be for example a nonwoven fabric.

Thin membranes of mineralized collagen have also been proposed in guided tissue regeneration applications, such as in US 6 417 166. Said mineralized collagen membrane comprises a substantially homogeneous mineralized collagen composite consisting essentially of 30 to 70% by weight of a collage component and 30 to 70% by weight of a calcium phosphate component precipitated from a collagen slurry by a soluble calcium ion-containing solution and a soluble phosphate ion-containing solution.

WO 2011/150482 discloses a method of obtaining resorbable composites based on bacterial cellulose and collagen for application in tissue repairing. In one embodiment, the composite additionally comprises hydroxyapatite. Similarly, WO 2010/001328 discloses a bioresorbable and flexible membrane comprising a biodegradable macromolecular fraction and an osteoconductive material such as hydroxyapatite. Said membrane exhibits asymmetric osteoconductive behaviors in both faces.

Hydroxyapatite is a naturally occurring mineral comprising calcium phosphates and has the formula Ca₅(PO₄)₃(OH). Calcium phosphates are biocompatible and osteoconductive, which means that newly formed bone tissue deposits directly thereon. In addition, some of them are bioresorbable, because they are recognized as body-consistent, i.e., the body recognizes them as part of itself, and they can be catabolized by specific bone-resorbing cells, such as osteoclasts, within the framework of natural bone metabolism and restructuring. During such restructuring processes, calcium phosphates can be catabolized and substituted by endogenous bone.

In US 2009/0131949 a cement preparation is disclosed which comprises calcium and strontium ions. Said preparation is a good bone substitute for osteoporotic bone.

Although a lot of preliminary investigations have been carried out, the stimulation of bone formation at early stages of state-of-the-art membranes is not satisfactory.

Thus, the problem to be solved by the present invention is to provide a membrane that stimulates bone formation, in particular at early stages.

The problem is solved by a bioresorbable membrane having a first surface area, an opposite second surface area and an inner part arranged between said first surface area and said second surface area, wherein the first surface area is intended to be directed towards an application site, and said membrane comprises cross-linked collagen fibers obtained from gelatin, characterized in that a strontium ion containing osteoconductive powder material is dispersed in at least said first surface area.

Further preferred embodiments are subject of the dependent claims.

The membrane according to the present invention has a thin and flat sheet-like structure. The membrane has a first surface area, an opposite second surface area and an inner part arranged between said first surface area and said second surface area. This means that one face of the inner part is completely covered by the first surface area and the other face of the inner part is completely covered by the second surface area. That is, the membrane has two outer faces, one is the first surface area and the second is the second surface area. The first and the second surface area have independently of each other a thickness of 3 to 49 %, preferably 10 to 49%, more preferably 20 to 49%, even more preferably 30 to 49%, most preferably 45% of the total thickness of the membrane. The first surface area is intended to be directed towards an application site.

Said membrane comprises cross-linked collagen fibers obtained from gelatin. In a preferred embodiment, the cross-linked collagen fibers are the main component of the membrane. The cross-linked collagen fibers have the function of giving the membrane a structure. Further, it provides a mechanical barrier which prevents undesirable connective tissue or other tissue from entering the defect site. Collagen fibers which may be used in the membrane according to the present invention are collage fibers from gelatins extracted, for example, from bones, tendons, skins, and combs of mammals and bird species, such as humans, cow, pig, horse, and fowl. Gelatin having a porcine origin is most commonly used. Such gelatin-based collagen fibers may be prepared by, for instance, being extracted from the foregoing animals, but in general, a commercially available product can be used. A method for extraction is not limited, and examples of the same include conventionally known acid treatment or alkali treatment. Preferably, the gelatin has a molecular weight of about 8'000 to 500'000, most preferably 150'000 to 200'000.

The membrane according to the present invention is different from a pure collagen membrane without cross-linking. They are generally weaker when soaked in water and show a higher degree of swelling. Furthermore, they tend to degrade faster. In contrast thereto, the membrane according to the present invention does not visibly swell after soaking with water or blood and has suitable elastic properties.

An application site within the context of the present invention may be a defect site, particularly an oral tissue defect, such as an oral bone defect. Oral tissue defects are, for example, intrabony defects around the natural tooth, dehiscence defects at the natural tooth (indication in periodontology), peri-implant defects, dehiscence defects at dental implants, deficient alveolar ridges, extraction sockets and pneumatized sinus (indications in implant dentistry).

At least in said first surface area of the membrane a strontium ion containing osteoconductive powder material is dispersed in the cross-linked collagen fibers obtained from gelatin by thermal denaturation. The cross-linked collagen fibers have primarily the function of a carrier. The strontium ion containing osteoconductive powder material is a material which enhances bone growth and comprises strontium cations. Said powder material may be homogenously dispersed within said first surface area. However, preferably the strontium ion concentration gradually decreases from the surface towards the inner part of the membrane, that is, the powder material is gradually dispersed in said first surface area.

Upon application of the membrane, the powder material degrades and releases strontium ions at the defect site. Due to the strontium ion containing osteoconductive powder material, the surface of the first surface area is rough, which additionally enhances the invasion of cells forming the desired tissue.

Due to the strontium ion containing osteoconductive powder material in said first surface area, the membrane according to the present invention induces an outstanding bone formation, especially within the first, for example four weeks, after implantation.

In a first step during the formation of bone, woven bone is produced, which is characterized by haphazard organization cf collagen fibers. Woven bone is mechanically weak. Later on, said woven bone is replaced by lamellar bone, which has a regular parallel alignment of collagen into sheets and is mechanically stable. Due to the strontium containing osteoconductive powder material in said first surface area, the regenerated bone has a more mature bony structure (higher degree of lamellar structure) compared to the site treated with the other membranes known in the state of the art, the newly formed bone of which has still a haphazard organization. Moreover, the membrane provides a mechanical barrier which prevents or inhibits undesirable tissue cells of the overlying tissue from proliferating into the defect site thereby allowing colonization of desired tissue cells for undisturbed healing and tissue regeneration. Particularly, it inhibits non desired tissue cells from entering the defect site, thereby supporting undisturbed healing of bone tissue (in implant dentistry) or periodontal tissue (in periodontology). At the same time, the membrane provides the structure upon or within which the desired bone or cartilage cells may grow in order to regenerate the desirable tissue.

In a preferred embodiment of the present invention, the strontium ion containing osteoconductive powder material comprises a strontium salt selected from the group of strontium orthophosphate (Sr₃(PO₄)₂), strontium hydrogen orthophosphate (SrHPO₄), strontium carbonate (SrCO₃), strontium chloride (SrCl₂), strontium hydroxyapatite (Sr₁₀ (PO₄)₆ (OH)₂ also known as Sr₈ (PO₄)₃ (OH)) or a mixture thereof. During degradation of the membrane, strontium ions are released and promote selective cell population, especially periodontal ligament cells and/or cementoblasts and/or osteogenic cells, by enabling cell adhesion and proliferation. Further, said strontium ions enhance restoration of tissue, such as oral bone, cementum and periodontal ligament as well as tissue maturation (especially bone maturation). Most preferred, the strontium salt is strontium hydroxyapatite (Sr₁₀(PO₄)(OH)₂) .

The osteoconductive powder material may comprise other osteoconductive salts, such as calcium phosphate (TCP) or apatite. However, the content of the strontium salts should be more than 25%, preferably more than 90 % of the total weight of the powder material. This also include strontium salt contents of 30%, 35%, 40%, 45%, 50%, 55%, 60%, 65%, 70%, 75%, 80% and 85% of the total weight of the osteoconductive powder material. Nevertheless, most preferred, the osteoconductive powder material comprises only strontium salts and no further csteoconductive salts.

In a preferred embodiment of the present invention, the osteoconductive powder material is a strontium-enriched hydroxyapatite or strontium hydroxyapatite. A strontium-enriched hydroxyapatite is a hydroxyapatite, wherein parts, preferably more than 35%, of the calcium ions have been replaced by strontium ions, whereas in Strontium hydroxyapatite all calcium ions have been replaced by strontium ions or the strontium hydroxyapatite has directly been produced in the absence of calcium ions. Membranes comprising a strontium-enriched hydroxyapatite have a positive effect on bone formation as well as an inhibitory effect on cellular bone resorption. Strontium hydroxylapatite and strontium enriched-hydroxyapatite are very easy to produce at low cost.

In another embodiment of the invention, not only the first surface area but also the inner part of the membrane comprises the strontium ion containing osteoconductive powder material. Said osteoconductive material may be dispersed only in a subarea of the inner part directly adjacent to the first surface area or it may be dispersed in the entire inner part. Such a membrane has a long term release characteristic, since the outer part of the membrane degrades first. As a result of the degradation, the strontium ions are released, i.e. they are in contact with the surrounding tissue. However, after some time, the strontium ion concentration will decrease due to solubilization and vascularization. According to this embodiment, the inner part of the membrane also contains strontium ions, which will then be released and continue to stimulate the osteointegration.

The second surface area may be essentially free of strontium ions cr may contain strontium ions. In case of only one application site, the second surface area comprises preferably only a low strontium ion concentration (that is less than 2.5%), or is most preferably, essentially free of strontium ions and acts mainly as barrier impermeable to undesirable cells. The surface of such a second surface area is smooth and is intended to be directed towards the tissue that covers the membrane after application.

In case of several application sites or large defects, the strontium containing ion osteoconductive powder mixture may also be dispersed in part of or in the entire second surface area. The surface of such a second surface area is intended to be directed towards the tissue that covers the membrane after application or towards a second defect site.

In a preferred embodiment of the present invention, the membrane is freeze dried. Such a membrane has no or only a small water content, that is less than 5% by weight. The freeze-dried membrane has the advantage, that it may be stored at room temperature for at least 6 months.

In another embodiment of the present invention, the concentration of the strontium ion containing osteoconductive powder material in the preferably freeze-dried membrane is at least 5%, preferably at least 10% and most preferably between 15 and 30% by weight of the total weight of the freeze died membrane. The membrane according to the present invention comprising between 20 and 30%, preferably between 25 and 30% of the strontium ion containing osteoconductive powder material has strong mechanical strength. Such a membrane is self-supportive and may be used without the support of avoid filler material, such as bone graft (substitute), applied underneath. If the membrane according to the present invention has a low strontium ion containing osteoconductive powder material concentration (i.e. between 5 and 20%) it is non-supportive and is to be used in combination with a void filler material, such as bone graft (substitute).

The size and shape of the particles of the osteoconductive powder material influences the distribution within the membrane. The size of the particle can be controlled by sieving. If the first surface area and the second surface area are intended to exhibit an asymmetric osteoconductive behavior, meaning that the first surface area has a high strontium ion concentration, whereas the second surface area has a low strontium ion concentration or is essentially free of strontium ions, the particles have preferably a size from 20 to 80 µm, most preferably from 25 to 60 µm. In contrast thereto, if the powder material is intended to be homogenously distributed within the entire membrane, the preferred particle size depends on the thickness of the membrane. For example, for a membrane having a thickness of 100 to 150 µm, the particles have preferably a size from 10 to 50 µm. Preferably, the osteoconductive powder material is amorphous, since it is obtained by precipitation. However, a hydrothermal treatment can be carried out to crystallize the particles. The degree of crystallization can further control the degradation of the strontium ion containing osteoconductive powder material.

In a preferred embodiment, the strontium ion concentration in the membrane according to the present invention gradually decreases from the first surface area towards the second surface area. Preferably more than 50%, most preferably more than 75%, and ideally 80 to 85% of the strontium ion containing osteoconductive powder material is within the first surface area. Preferably, the strontium ion containing osteoconductive powder material forms at the very outer part of the first surface area an almost homogenous layer so that no cross-linked collagen fibers can be seen. That means that also within the first surface area there is a strong concentration gradient.

During the freeze dry step, that is, when eliminating the water during the production of the membrane, the particles of the strontium ion containing osteoconductive powder material are deposited towards one of the faces of the membrane, that is, the first surface area. This process is mainly induced by gravity. The gradient in the concentration of the particles is the result of the combination of the rate of jellification of the collagen fibers obtained from gelatin by thermal denaturaticn and the speed of deposition of the powder mixture towards one of the faces of the membrane. The speed of deposition of the powder mixture is mainly influenced by the particle size and shape.

In another embodiment, the strontium ion containing osteoconductive powder material is homogenously distributed within the whole membrane. That is, the first surface area, the inner part of the membrane and the second surface area have all essentially the same concentration of the strontium ion containing osteoconductive powder material. This results in a constant release of the strontium ions over a longer time period.

In a preferred embodiment, the gelatin which is used as starting material for the cross-linked collagen fibers has an isoelectric point from 4.5 to 5.5, preferably 4.7 to 5.3, most preferably 5.1. Preferably, a gelatin type B is used, which is isolated using an alkaline process. The isoelectric point of the gelatin has an influence on the crosslinking of the collagen fibers. Further, it has been shown, that membrane with cross-linked collagen fibers obtained from such a gelatin may have a strong affinity with many growth factors such as FGF, PDGF and VEGF, which are important proteins for bone growth. Therefore, most probably the membrane according to the present invention does not only maintain the space for bone formation, but also acts as fine reservoir for endogenous growth factors.

The thickness of the freeze dried membrane according to the present invention can be designed according to, for instance, the application site and/or the desired degradation time of the cross-linked collagen fibers obtained from gelatin. It is preferably less than 500 µm, most preferably in the range of 100 µm to 150 µm.

Collagen fibers obtained from gelatin is biocompatible and biodegradable and, therefore, the membrane of the present invention is also biodegradable. Though the degradation time of the membrane varies with the application site and is dependent on the membrane thickness as well as the cross-linking and the molecular weight of the collagen fibers, the membrane of the present invention preferably is degraded in up to six months, preferably in up to three months.

Preferably, the membrane according to the present invention does not comprise further substances. However, it is possible to incorporate drugs, such as antibiotics. In such case, the drugs will be preferably added to the solution comprising gelatin and the powder mixture before its processing into the membrane form.

Preferably, the membrane according to the present invention has a sheet form.

In a preferred embodiment, the strontium ion containing powder material is a strontium hydroxyapatite Sr₁₀(PO₄)₆(OH)₂. Strontium hydroxyapatite may be obtained by mixing a strontium salt, preferably strontium chloride, and a phosphate source, preferably disodium hydrogen phosphate, in water. The pH of the solution is adjusted to a pH above 7, preferably a pH from 8.5 to 9.5, most preferred a pH of 9 resulting in the precipitation of essentially pure strontium hydroxyapatite.

The membrane according to the present invention may be prepared as follows: In a first step, an aqueous solution comprising completely dissolved gelatin is prepared. Typically, the gelatin is dissolved by heat treatment at 60°C for 1 hour. In a second step, the strontium ion containing powder osteoconductive powder material is added to the solution obtained in the first step and then, they are mixed together. Said mixture is poured in a third step on a flat surface, such as a petri dish, a polystyrene plate or the like. Finally, the mixture is dried under constant air for 5 to 10 hours, resulting in a precursor-membrane. The precursor-membrane may be cut into the predetermined size in order to obtain the desired contour and size that are adequate for the specific application. Afterwards, the precursor-membrane is subjected to freeze-drying at -50°C to -80°C, preferably overnight. Finally, the freeze-dried membrane is crosslinked by heat at at least 60°C, which results in intermolecular crosslinking of the collagen fibers. Preferably, the thermal crosslinking is carried out at 140°C to 180°C, most preferably at 160°C. A preferred treatment time is more than 4 hours, most preferred 6 hours. Due to the crosslinking the mechanical strength as well as the degradation resistency of the membrane increases. Moreover, after soaking with water or blood, such a cross-linked membrane does not have the tendency to swell. Optionally before carrying out the freeze-drying step the mebranes may be allowed to swell within distilled water at 4°C for 1 hour. Further the distilled water may be removed and the membranes may optionally be kept in a deep-freezer (-10°C) for 10 minutes, which can help to maintain a porous structure within the membrane, so that the cells can grow in.

After production, the membrane may be sterilized and packed. The membrane may be sterilized, for example, with gamma radiation or ethylene oxide. Most of the operations should be carried out in sterile conditions, using, for example, a clean room.

The membrane according to the present invention has many medical applications because of its flexibility, strong mechanical strength, easy manipulation character, excellent biocompability and controllable bioresorption rate. Such medical applications include tissue repairing, especially if said tissue is a bone or a periodontal tissue, as well as surgical treatments cf oral tissue defects. Further it may be used as a membrane barrier for guided tissue regeneration applications for repairing periodontal defects, or as a membrane for covering bone defect surgery and for bone substitutes (such as filler material). It may also be used for humans with impaired bone healing pattern, such as osteoporosis, diabetes mellitus and heavy smokers.

As an illustrative example, the membranes according to the present invention may be applied following the procedure below: The membrane is shaped to the desired shape for *in situ* adaptation. Afterwards, the membrane is placed on the application site, for example, on top of the void filler material with the first surface area facing the defect area. Preferably, the membrane overlaps the defect border in order to make sure that it does not sink into the defect. The membrane may be applied in parallel to dental implant placement. The membrane is typically applied after insertion of the dental implant. The membrane is moistened with a solution, such as a saline solution or blood retrieved from the defect site. There is no need of any direct fixation. However, the membranes may also be fixed using sutures, (resorbable) pins or surgical adhesives such as fibrin glue.

### Figures:

- Figure 1: shows a cross-sectional view of the membrane according to the present invention.
- Figure 2: shows a diagram relating to the new bone formation.
- Figure 3: shows a diagram relating to the bone volume (mm³) .

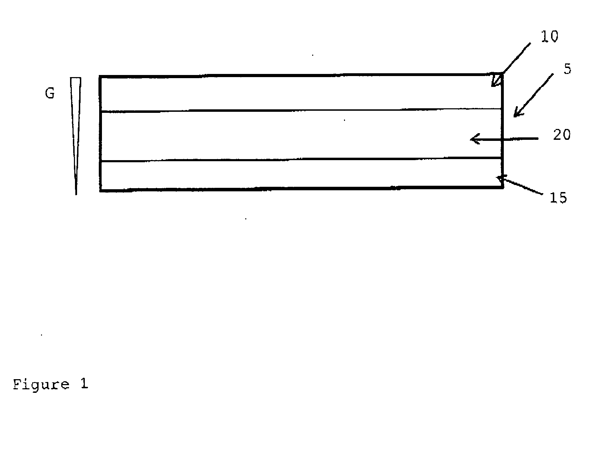

Figure 1 shows a schematic cross-sectional view of the membrane (5) according to the present invention. The membrane (5) has a first surface area (10), an opposite second surface area (15) and an inner part (20) arranged between said first surface area (10) and said second surface area (15). The first surface area (10) is intended to be directed towards an application site. At least in said first surface area (10) the strontium ion containing ostecconductive powder material is dispersed in the cross-linked collagen fibers obtained from gelatin. In a preferred embodiment of the invention, the strontium ion concentration gradually decreases from the first surface area towards the second surface area (shown as G in figure 1). This gradual decrease may be continuous or stepwise (not shown in Figure 1). Such stepwise decrease may be realized by assembling layers with different strontium ion concentrations.

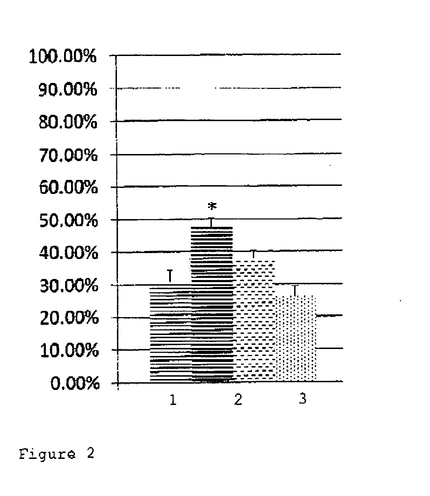

Figure 2 shows the values of total defect area and area of newly formed bone measured with imageJ software. The bone fill percentage (%) to the total defect was calculated. In the x-axis of the diagram 1 stands for the animal group wherein the defect was covered by the membrane according to example 3 (see below), 2 stands for the animal group wherein the defect was covered by the membrane according to example 4 (see below), 3 stands for the animal group wherein the defect was covered by a collagen membrane and 4 stands for the animal group, wherein the defect was not covered (left empty) as control. The asterisk * stands for p < 0.05 vs. group 1, group 3 and group 4.

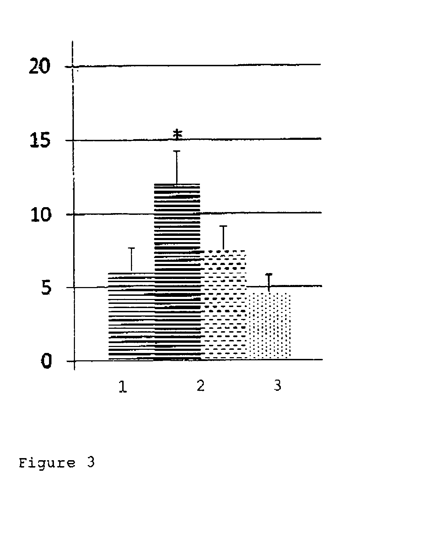

Figure 3 shows the bone volume in the defects (in mm³) at 4 weeks measured by micro-CT. In the x-axis of the diagram 1 stands for the animal group wherein the defect was covered by the membrane according to example 3 (see below), 2 stands for the animal group wherein the defect was covered by the membrane according to example 4 (see below), 3 stands for the animal group wherein the defect was covered by a collagen membrane and 4 stands for the animal group, wherein the defect was not covered (left empty) as control. The asterisk * stands for p < 0.05 vs. group 1, group 3 and group 4 (one way Anova, sheffe test).

### Examples:

### Example 1: Preparation of a strontium ion containing powder mixture

SrCl₂ (0.5M) (Wako, Japan) and Na₂HPO₄ (0.3M) (Sigma, Japan) are dissolved with 700 ml distilled water inside a 1000 ml beaker under constant stirring. A 10% aqueous ammonium solution (Sigma, Japan) is gradually added to maintain a stable final pH of 9. Distilled water is added to reach a final volume of 1000 ml. The resulting precipitate is collected and washed with 80°C hot water three times (with the aid a of centrifugator) to remove NaCl contamination. The precipitate is collected and dried in a clean bench under constant air blow for 8 hours. The dried powder is sieved through a 45 µm sieve mesh obtaining essentially pure strontium hydroxyapatite (Sr₁₀(PO₄)₆)OH)₂ having essentially the same particle size.

### Example 2: Collagen fibers obtained from gelatin

Gelatin blocks (MediGelatin) supplied by Nippi, Incorporated, Japan, are washed and soaked into distilled water to have the concentration of 5% (weight). The gelatin blocks are allowed to swell at room temperature for one hour. Thereafter, they are incubated at 60°C for one hour.

### Example 3: Membrane preparation and cross-linking of collagen fibers obtained from gelatin

60 mg of the strontium hydroxyapatite powder mixture prepared in Example 1 are mixed with the 6 ml collagen fibers obtained from gelatin solution in step 2 at 60°C. 3 ml of the above mixture is poured into 60 mm petri dish at 40°C, whereby the liquid depth is around 1.5 mm. This mixture is dried inside a clean bench under constant air for 8 hours which will generate a precursor-membrane. The precursor-membrane is cut into a predetermined size and can be collected from the petri dish. Thereafter, the precursor-membranes are allowed to swell within 4°C distilled water for 1 hour. Afterwards the distilled water is removed and the precursor membranes are kept in a deep-freezer (-70°C) for 10 minutes. The precursor-membranes are subjected to freeze drying overnight in order to remove the water from the membrane. Finally, the freeze-dried membrane is crosslinked by heat at 16C°C for 6 hours. The obtained membrane comprises 16.5% strontium hydroxyapatite.

### Example 4:

Same procedure as in Example 3. Instead of 60 mg, 120 mg of the powder mixture are mixed with the 6 ml collagen fibers obtained from gelatin solution in step 2 at 60°C. The obtained membrane comprises 28.6% strontium hydroxyapatite.

### Example 5:

Bilateral 5 mm calvarial defects are created in adult male Wistar rats and covered by the membranes according to Example 3 or Example 4, respectively, and compared with a Koken Tissue Guide membrane which is a commercially available collagen membrane. Said membrane consists of native collagen proteins after protease treatment to cleave away the ends of the collagen (atelocollagen). In cne group the defect is empty as negative control. The animals are sacrificed at 4 weeks and analyzed radiologically and histologically.
- Group 1: Defect covered by the membrane according to example 3.
- Group 2: Defect covered by the membrane according to example 4.
- Group 3: Defect covered by the collagen membrane.
- Group 4: Defect not covered (left empty).

At 4 weeks, both micro-CT and histological analysis reveal that the membranes having a concentration of 20 mg/ml of the strontium ion containing osteoconductive powder material yield significantly greater bone formation than the other groups (Figure 2: The values of total defect area and area of newly formed bone are measured with ImageJ software, and the bone fill percentage (%) to the total defect is calculated). In addition, in group 3 (defect covered by the collagen membrane) a large part of the new build bone is still woven bone, which is characterized by haphazard organization of collagen fibers. In contrast thereto, in group 2 (defect covered by a membrane according to example 4) a significant part of the woven bone has already been replaced by lamellar bone. The regular parallel alignment of collagen into sheets is clearly detectable resulting in a mechanically stable bone. (Figure 3: Bone volume in the defects at 4 weeks measured by micro-CT), The present results indicate that the membrane according to the present invention stimulates bone formation at early stages.

## Claims

1. A bioresorbable membrane having a first surface area, at opposite second surface area and an inner part arranged between said first surface area and said second surface area, wherein the first surface area is intended to be directed towards an application site, and said membrane comprises cross-linked collagen fibers obtained from gelatin, **characterized in that**
a strontium ion containing osteoconductive powder material is dispersed in at least said first surface area.

2. Membrane according to claim 1, **characterized in that** the strontium ion containing osteoconductive powder material comprises a strontium salt selected from the group of strontium orthophosphate, strontium hydrogen orthophosphate, Strontium carbonate, strontium chloride, strontium hydroxyapatite, or a mixture thereof.

3. Membrane according to any of the preceding claims, wherein the strontium ion containing osteoconductive powder material is a strontium hydroxyapatite or a strontium-enriched hydroxyapatite.

4. Membrane according to any of the preceding claims, **characterized in that** the strontium ion containing osteoconductive powder material is additionally dispersed in the inner part of the membrane.

5. Membrane according to claim 4, **characterized in that** the strontium ion containing osteoconductive powder material is additionally dispersed in the second surface area.

6. Membrane according to any of the preceding claims, wherein the membrane is freeze-dried.

7. Membrane according to claim 6, wherein the concentration of the strontium ion containing osteoconductive powder material in the freeze-dried membrane is at least 5%, preferably at least 10%, most preferably between 15 and 30% by weight of the freeze-dried membrane.

8. Membrane according to any of the preceding claims, wherein the concentration of the strontium ion containing osteoconductive powder material gradually decreases from the first surface area towards the second surface area.

9. Membrane according to any of claims 1 to 7, wherein the strontium ion containing osteoconductive powder material is homogeneously distributed within the entire membrane.

10. Membrane according to any of the preceding claims, **characterized in that** the gelatin has an isoelectric point from 4.5 to 5.5, most preferably about 5.1.

11. Membrane according to any of claims 6 to 10, wherein the freeze dried membrane has a thickness of less than 500 µm, preferably 100 µm to 150 µm.

12. Membrane according to claim 1, wherein the membrane is in a sheet form.

13. Method for preparing a membrane according to claims 1 to 12 by
a) preparing an aqueous solution comprising completely dissolved collagen fibers obtained from gelatin,
b) adding the strontium ion containing osteoconductive powder material to the dissolved collagen fibers obtained from gelatin solution obtained in step a) and mixing them together,
c) pouring the mixture obtained in step b) on a flat surface,
d) drying the mixture under constant air for 5 to 10 hours, resulting in a precursor-membrane,
e) freeze-drying said precursor-membrane, preferably overnight,
f) cross-linking the freeze-dried membrane obtained in step e) by heat, preferably at 140°C to 180°C, most preferably at 160°C.

14. Use of a membrane according to any of claims 1 to 12, wherein said membrane is used for tissue repairing, as barrier membrane or as membrane for covering tissue defects.

15. Use according to claim 14, wherein said tissue is an oral tissue, such as bone, cementum and periodontal ligament.
